(19) Europäisches Patentamt

European Patent Office

Office européen des brevets

(11) Publication number: **0 558 685 B1**

(12) **EUROPEAN PATENT SPECIFICATION**

(45) Date of publication of patent specification: **06.09.95**　(51) Int. Cl.⁶: **C09J 7/02**, C09J 153/00, A61F 13/60

(21) Application number: **92903003.9**

(22) Date of filing: **15.10.91**

(86) International application number: **PCT/US91/07718**

(87) International publication number: **WO 92/08763 (29.05.92 92/12)**

(54) **FRONTAL TAPE-BASED DIAPER CLOSURE SYSTEM.**

(30) Priority: **20.11.90 US 616226**

(43) Date of publication of application:
**08.09.93 Bulletin 93/36**

(45) Publication of the grant of the patent:
**06.09.95 Bulletin 95/36**

(84) Designated Contracting States:
**BE DE ES FR GB IT NL SE**

(56) References cited:
**EP-A- 0 249 461**
**EP-A- 0 306 232**
**US-A- 4 780 367**

(73) Proprietor: **MINNESOTA MINING AND MANU-FACTURING COMPANY**
**3M Center,**
**P.O. Box 33427**
**St. Paul,**
**Minnesota 55133-3427 (US)**

(72) Inventor: **MILLER, John, A.**
**Post Office Box 33427**
**Saint Paul, MN 55133-3427 (US)**
Inventor: **TATE, Earl, Jr.**
**Post Office Box 33427**
**Saint Paul, MN 55133-3427 (US)**
Inventor: **VALASOUEZ UREY, Ruben, E.**
**Post Office Box 33427**
**Saint Paul, MN 55133-3427 (US)**

(74) Representative: **Baillie, Iain Cameron et al**
**c/o Ladas & Parry**
**Altheimer Eck 2**
**D-80331 München (DE)**

**Description**

Background and Field of the Invention

This invention relates to an improved pressure-sensitive adhesive for use with adhesive diaper closure tapes and the like, and more specifically, to a pressure-sensitive adhesive composition which provides improved performance characteristics when used in a diaper closure system having an adhesion surface treated with a release agent.

European Patent Application No. 306232 discloses a diaper tape closure system comprising a pressure-sensitive adhesive fastening tape designed to refastenably adhere to a diaper backsheet (e.g., a thin polyethylene film) characterized in that the pressure-sensitive adhesive is an ABA block copolymer tackified with a liquid resin, a solid resin and an end block reinforcing resin. The tape exhibits a maximum peel force between peel rates of 10 cm. per minute and 400 cm. per minute.

European Patent Application No. 249461 discloses dispensable medical tapes coated with a pressure-sensitive adhesive, such as tackified ABA block copolymers, on one face characterized in that the opposite face is coated with a low adhesion backsize and an elastomer in a one-pass coating operation.

There are numerous patents and literature documents that are directed to the use of block copolymers in adhesive compositions which traditionally include a block copolymer such as an ABA block copolymer and a tackifying resin. U.S. Patent No. 3,427,269 (Davis), describes the use of an elastomeric based adhesive comprising at least 100 parts of an ABA block copolymer, a rosin ester (10-50 parts) and a coumarone-indene resin (25-50 parts). However, as development of these elastomer based compositions has progressed it has been found that the adhesive properties of these block copolymer based adhesive compositions are extremely sensitive to the particular block copolymer(s) used, and the proportion and type of modifiers such as solid or liquid tackifiers, aromatic or aliphatic tackifiers, plasticizers, extender oils, curing agents and the like.

Korpman, U.S. Patent Nos. 4,080,348 and 4,136,071, describe pressure-sensitive adhesives based on polystyrene-polyisoprene-polystryrene ABA block copolymers. The patents claim adhesives having varying percentages of diblock and triblock (AB and ABA) copolymers. The 4,136,071 patent claims adhesives with 50% to 90% triblock, and the remainder diblock, with a suitable tackifying resin. These adhesives are alleged to have good anchorage properties to the tape backing as well as excellent fiber wetting on boxboard. The 4,080,348 patent claims adhesive having 15% to 45% triblock, with the remainder diblock copolymers, with a suitable tackifying resin. The claims are also limited to certain percentages of polystyrene in the block copolymers. The adhesives of this patent are alleged to have superior adhesion to skin.

U.S. Patent No. 3,932,328 (Korpman) describes the use of a prior art adhesive based on an ABA block copolymer with a solid tackifying resin and an extender oil. He describes this composition as unsatisfactory as a pressure-sensitive adhesive(PSA) and proposes a specific composition comprising a styrene-isoprene-styrene (SIS) copolymer and specific proportions of an aliphatic solid tackifier and a liquid tackifier (at 25°C). Korpman reports that his adhesive had "satisfactory" adhesion and quick-stick for most applications. The prior art patent Korpman was referring to was U.S Patent No. 3,239,478 (Harlan) who discloses both PSA and non-PSA ABA block copolymer based adhesives. Harlan only exemplifies a PSA formed of 100 parts of a SIS block copolymer (48 percent S), 75 parts of a 2% aromatic extender oil and 125 parts of a tackifying resin of glycerol ester of polymerized rosin.

U.S. Patent No. 3,954,692 (Downey) also addressed the problems with ABA based adhesive compositions, particularly hot melt pressure-sensitive adhesive compositions, particularly problems with providing adequate adhesion and strength properties. Downey et al. was particularly concerned with providing a general-use, pressure-sensitive adhesive with not only adequate tack but good peel strength and good shear strength. Downey et al. proposed an SIS block copolymer admixed with conventional extender oils, which were stated as unsatisfactory by Korpman, using a specific tackifying resin. The resin exemplified comprised a polymerized admixture of piperylene and 2-methyl-2-butene, which is currently marketed, e.g., as Wingtack™ 95, available from Goodyear Chemical Company.

An alleged improvement, for an adhesive designed to be used with porous substrates (e.g., Kraft paper), on the Downey et al. formulation is proposed by U.S. Patent No. 4,097,434 (Coker) which describes conventional practice as using extending oils with low aromatic content in formulations in which extending oils are employed. Coker states that these formulations have inadequate holding power to porous substrates and proposes a formulation of an ABA copolymer (only SIS is exemplified) with a tackifying resin (only the Downey et al. Wingtack™ 95 is exemplified) and an extending oil with a high aromatic content (above 55%) and a saturates content of less than 15%. The two oils compared were Shellflex™ 371 (non-invention) and

2

Dutrex™ 739 (as per the claimed invention). The Shellflex™ 371 formulations were nominally presented to show formulations with inadequate adhesion properties.

Another variation is proposed in U.S. Patent No. 4,460,364 (Chen et al.) which describes a composition having certain rheological properties that they propose are indicative of ABA based adhesives with tenacious bonding and clean release, where the B block is a polyolefin (only polystyrene-polyethylene polybutylene-polystyrene exemplified). The SEBS copolymers exemplified are used with a solid tackifying resin and a plasticizing or extending oil. Chen et al. states that these compositions do not sacrifice clean release for tenacity as is stated is typically done in prior art hot melt PSAs. Chen et al. was primarily concerned with an adhesive which could be used in a sanitary napkin without leaving residue adhesive on the undergarment. This rheological approach is also proposed by U.S. Patent No. 4,719,261 (Bunnelle et al.) who uses the same rheological properties to define a hot melt PSA that allegedly can also be used as an elastic. The specific compositions claimed comprise specific ABA (i.e., SIS) copolymers (45-75%) with both aromatic (2-9%) and aliphatic (25-50%) solid resins.

U.S. Patent No. 3,935,338 (Robertson et al.) describes an adhesive composition that allegedly has a low enough viscosity to be applied as a hot melt and will have enhanced PSA properties at elevated temperatures. The formulation exemplified as meeting this requirement is an SIS block copolymer (100 parts) with an elastomer block compatible resin (50-200 parts), a reinforcing resin (25-100 parts) and an extending oil (25-100 parts). The patent also discloses compositions excluding the reinforcing resin as counterexample formulations that have inadequate adhesive properties at elevated temperatures.

Korpman has also proposed modifications to his formulations, and approach; U.S. Patent No. 4,813,947 (use of a ABA(SIS) and a tackifier which is coupled with a specific hot melt landing area); and 4,540,415 (use of a SIS a solid tackifier and a phosphatide).

Although there exists extensive art on the use of block copolymers in PSA compositions, due to the uncertainty in results and continuing need, the search continues for adhesive formulations with yet improved mixes of properties such as adhesion characteristics, such as shear strength and peel strength. Particularly, there is the continuing need for the identification of adhesive formulations that have a proper mix of adhesive properties such as would make them particularly suitable for use in diaper closure systems. The engineering of diapers is highly specialized. Adhesive closure systems used in this environment ideally should have a particular set of properties.

## Summary of the Invention

It has been found that a hot melt pressure-sensitive adhesive can be provided which has advantageous adhesion and cohesive properties when used on a fastening tab in a diaper closure system having a release treated adhesion surface which adhesive composition comprises:

(a) 30 to 60 weight percent of an elastomeric A-B (A) block copolymer of at least one polystyrene block A and at least one polyisoprene block B, wherein the A blocks comprise from 10% to 30% of the copolymer and at least 25 percent of said AB block copolymer comprises an AB diblock copolymer with the balance comprising an ABA block copolymer, and

(b) a mixture of solid tackifying resin, liquid tackifying resin and/or plasticizing oil so as to provide a composite midblock glass transition temperature of from about 264 to 244 Kelvin,

which adhesive composition exhibits a nonshocky peel and a 135° peel strength of greater than approximately 120 grams per cm [300 grams per inch] (as defined herein) to release coated substrate, and a shear strength of at least 100 minutes (as defined herein) to the same substrate.

This composition is described in a cartesian space system where the CMTg values are on the x-axis and the percent elastomer is on the y-axis.

## Brief Description of the Drawing

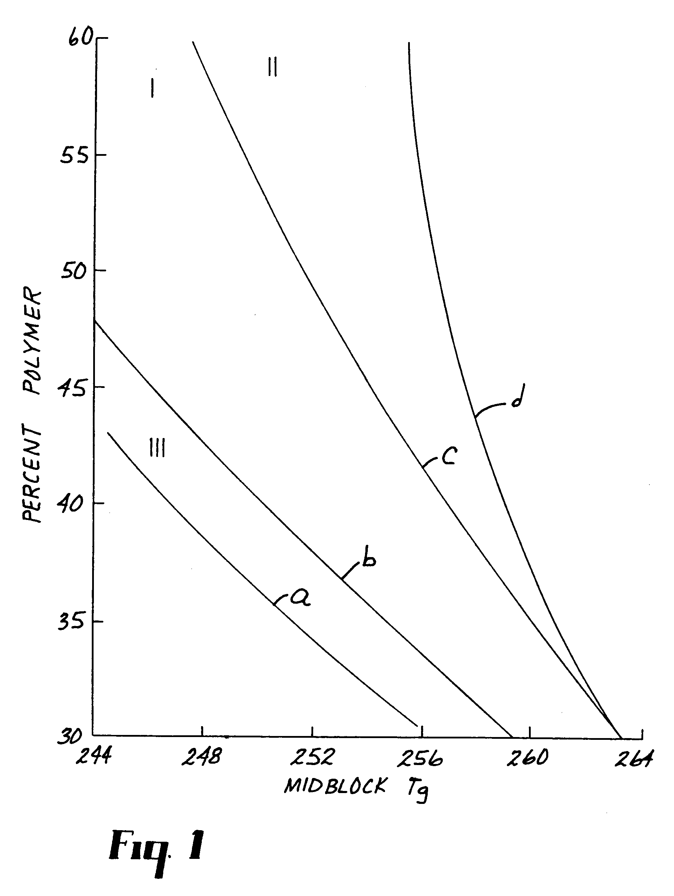

Fig. 1 is a diagram outlining the preferred adhesive compositions based on percent elastomer and CMTg.

## Detailed Description of the Preferred Embodiments

The diaper closure system of the invention can be used with any conventional diaper which uses an adhesive fastening tab to effect closure of the baby diaper or adult incontinence device, by attachment to a Low Adhesion Backsize (LAB) treated polyolefin or like film. The film is generally a reinforcing film, typically attached to a liquid-impermeable outer shell of the diaper. Many diapers are provided with a reinforced area where the tape adheres to the diaper. These reinforcing areas are generally formed of a relatively stiff polymer film, such as a polypropylene film, which is generally attached directly to a liquid-impermeable outer shell film. These reinforcing films are typically supplied as a tape in a roll form with the adhesive preapplied. In order to unroll the tape from the roll the tapes are provided with what is termed a Low Adhesion Backsize (LAB) by chemical or like treatment. A typical LAB treatment is a urethane coating. However, reinforcing film cut from these LAB-treated tapes complicates adhesive diaper closure systems by interfering with subsequent adhesion of the fastening tab, making the selection of the appropriate adhesive to use on the fastening tab difficult.

Two important aspects of fastening tab performance in a diaper, or the like, adhesive closure system are the shear resistance and peel strength and performance. Peel strength is important in terms of adhesive fastening tape performance and customer perception of performance. A low peel strength bond increases the risk of popping open when subjected to the forces encountered during use. Further, low peel strengths are often associated with shocky peels (generally tested at a peel rate of 30 cm. per minute, 12 inches per minute). Shocky peels are well understood in the art and are when the tape peels in a jerky and noisy (sounding somewhat like a zipper) manner. The adhesive fastening tab of this invention exhibits consistently high peel values to LAB treated substrates, e.g., at least about 120 grams per cm. (300 grams per inch) to a common urethane LAB treated polyolefin film. Further, the invention adhesive fastening tape with its high elastomeric diblock component has consistently exhibited nonshocky peels to these LAB treated polyolefin films. These consistent, substantially non-shocky peels are extremely advantageous in an adhesive closure system. Peel forces much in excess of 300 gram per cm. (750 grams per inch) can be perceived as too high by some end users (this depends somewhat on the user). The high peels obtainable with the invention adhesive tapes occasionally exceed 300 grams per cm., however, not excessively such as to be perceived as a problem. Overall, the high substantially non-shocky peels are advantageous to a tape closure system as described.

The shear force resistance for a commercial adhesive fastening tab is preferably at least 200 minutes and more preferably at least 300 minutes with a 1 kilogram weight. Shear resistances of less than 300 down to about 100 are still nominally functional yet are not commercially desirable. Shear values much greater than 500 contribute little added functional or commercial benefit to a diaper fastening tab.

The preferred adhesive coating thicknesses on the fastening tabs range from 20 to 75 microns, preferably from 25 to 50 microns. With too thin of an adhesive layer thickness, the adhesion properties will be adversely affected, whereas excess coating thicknesses can be wasteful.

The block copolymers employed in adhesive compositions for the invention fastening tabs are thermoplastic block copolymers having a linear configuration and having the A blocks and B blocks formed in what are termed ABA block copolymers and AB block copolymers. The ABA block copolymer comprises 75 percent or less of the elastomer component, preferably from 35 to 70 percent; the AB block copolymer comprises at least about 25 percent of the elastomer component, preferably from 30 to 65 percent; the A block is a monoalkenyl arene, mainly polystyrene, having a molecular weight between 4,000 and 50,000, preferably between 7,000 and 30,000. The A block content is from about 10 to 50 percent, more preferably between 10 and 30 percent. Other suitable A blocks may be formed from alphamethyl styrene, t-butyl styrene and other ring alkylated styrenes as well as mixtures thereof. B is an elastomeric conjugated diene, namely isoprene, having an average molecular weight of from about 5,000 to about 500,000, preferably from about 50,000 to 200,000. Although preferably ABA and AB block copolymers will comprise the majority of the elastomer of the adhesive, other conventional diene elastomers may be used to a minor extent, such as natural rubber; butadiene, isoprene or butadiene-styrene rubber; butadiene-acrylonitrile; butyl rubber or block copolymers of these diene elastomers. The block copolymer is used in an amount ranging from about 30 to 60 weight percent, preferably at least 35 weight percent of the adhesive composition.

The tackifying resin component generally comprises a blend of a solid tackifying resin and a liquid tackifying resin, a single solid or liquid tackifying resin, or a blend of a solid tackifying resin and a liquid plasticizer and/or liquid tackifying resin. The tackifying resins can be selected from the group of resins at least partially compatible with the B blocks of the elastomeric materials of this invention. Such tackifying resins include those aliphatic hydrocarbon resins made from the polymerization of a feed stream consisting mainly of unsaturated species containing four to six carbon atoms; rosin esters and rosin acids; mixed aliphatic/aromatic tackifying resins; polyterpene tackifiers; and hydrogenated tackifying resins. The hydrogenated resins can include resins made from the polymerization and subsequent hydrogenation of a feedstock consisting mostly of dicyclopentadiene; resins produced from the polymerization and subsequent hydrogenation of pure aromatic feedstocks such as styrene, alphamethylstyrene, vinyl toluene; resins fashioned from the polymerization and subsequent hydrogenation of an unsaturated aromatic feedstream wherein the feedstream mainly contains species having from 7 to 10 carbon atoms; hydrogenated polyterpene resins; and hydrogenated aliphatic and aliphatic/aromatic resins. Preferred tackifying resins include the aliphatic hydrocarbon resins and the hydrogenated resins. Especially preferred are the aliphatic hydrocarbon resins.

The liquid plasticizers suitable for use in the adhesive compositions of this invention include naphthenic oils, paraffinic oils, aromatic oils, and mineral oils. Preferred plasticizing liquids include naphthenic oils and slightly aromatic oils.

The adhesive preferably is tackified with a solid tackifying resin with a liquid plasticizer or liquid resin of the above described preferred types.

Preferably, the solid tackifying resin used is one that is compatible with the elastomeric conjugated diene block and is preferably a tackifying resin having a softening point between 80° and 115°C, such as is produced from polymerization of a stream of aliphatic petroleum derivatives of dienes and monoolefins having 4 to 9 carbon atoms as is disclosed in U.S. Patent Nos. 3,939,328 and 3,954,692, the substance of which are incorporated herein by reference. Particularly preferred are tackifying resins resulting from the copolymerization of a feed comprised predominately of $C_5$ carbon atom species such as piperylene and 2-methyl-2-butene or isoprene, commercially available, for example, as Wingtack™ 95 and Wingtack™ Plus, respectively, from Goodyear Chemical Co.

The adhesive compositions can also be modified with well known additives such as pigments, fillers, stabilizers and antioxidants for their conventional purposes.

The fastening tab is formed by placing the adhesive described above on a conventional substrate. The fastening tab substrate can suitably be formed of a synthetic polymer such as polyolefins (e.g., polypropylene), polyesters, polyamides or the like. Natural backings such as Kraft paper backings may also be used. The adhesive can be applied by any conventional method including melt coating, gravure, coextrusion, solvent coating and the like.

The CMTg can be calculated using the Fox Equation from measuring the Tg of the midblock of the elastomeric block copolymer and the measured Tg of each tackifying resin and liquid plasticizer oil. The Tg for each component is measured using a differential scanning calorimeter such as a DSC-7, manufactured by Perkin-Elmer. The Tg is measured on the second heating run using a scan rate of 20 degrees Centigrade per minute. The first heating run is made up to well above the softening point of the test material. The sample is subsequently quenched to well below the Tg of the material. Antioxidants added to the adhesive are not figured into the calculation of the CMTg. The Fox Equation is:

$$\frac{\Sigma_i W_i}{CMTg} = \Sigma_i \frac{W_i}{Tg_i}$$

where $W_i$ is the weight fraction of component i and $Tg_i$ is the glass transition temperature of component i. Only the midblock portion of the block copolymer is included in the calculation of the CMTg. For a styrene/isoprene block copolymer, the midblock is the polyisoprene portion of the molecule.

The adhesive fastening tab is used in conjunction with a release tape where the fastening end of the fastening tab is placed prior to placement on the frontal reinforcing film or tape. The release tape can be of conventional design. One side will have a pressure-sensitive adhesive coating for adhering it preferably to the nonwoven inner liner of the diaper. The opposing side will be coated with a release agent such as crosslinked poly(dimethyl-siloxane).

The fastening tab of the invention is designed for use with frontal tapes having low adhesion backsize coatings such as an urethane, as is described in U.S. Pat. No. 2,532,011 (Dahlquist et al.). These frontal tapes, as described above, serve as the attachment surfaces for the fastening tab when the diaper, or the like, is in use.

Two conventional surface textures on a frontal tape are smooth surfaces such as is obtained with a biaxially oriented polypropylene or textured surfaces as is obtained with a matte-cast film. It has been found that different ranges of the invention fastening tab adhesive compositions work with the two different surface textures. These different compositional ranges are depicted in Fig. 1.

There are three compositional spaces depicted in Fig. 1 that being I, II, and III. These spaces are bounded on the bottom by compositions with 30 percent elastomer and at the top by compositions with 60 percent elastomer. The regions I and II are bounded at the left by compositions with CMTg's of greater than 244 Kelvin. Those adhesive compositions falling within region I have been found to provide superior performance as to both types of LAB coated frontal tapes (both smooth and matte finish). Namely, region I compositions provide smooth, or substantially non-shocky, peels, high peel strengths, and good or adequate shear resistance performance with the preferred tackifiers. For smooth frontal reinforcing films, preferred region I also extends into region III. However, region II compositions, although the peels are generally smooth, generally do not exhibit preferred levels of shear to smooth LAB-coated films. Conversely, for matte LAB-coated frontal reinforcing films, the preferred region I extends into region II. The region III compositions again show smooth and high peels to the matte film, however, do not necessarily have adequate shear resistance to standard matte surface textures.

The above described behavior depicted in Fig. 1 is altered with higher diblock content elastomers (e.g., greater than 45 percent). These higher diblock elastomer-based adhesives exhibit improved nonshocky peel at higher CMTg values, for a given percent elastomer. As such, the upper limit on peel performance with respect to the described LAB-coated substrates (defined by lines c and d) will shift to the right for higher elastomeric diblock-containing adhesive compositions.

The CMTg values described above, although an excellent predictor of peel behavior for a given percent diblock adhesive composition, may not adequately predict shear performance with respect to certain non-preferred tackifiers and tackifying systems. For these systems, shear can be raised within the teachings of this invention by increasing the elastomer and/or solid resin content of the adhesive composition used within the outlined CMTg ranges.

A further aspect of the adhesive formulation used is, it is generally suited to hot-melt coating techniques, which is advantageous in terms of environmental impact.

The following examples are the currently contemplated preferred modes for carrying out the invention and should not be considered as limiting thereof unless otherwise indicated.

Examples

The samples were prepared by coating the adhesives as listed in Table 1 onto cast polypropylene films, exhibiting a matte finish. For the examples, the polypropylene film was 4 mils (100 microns) thick and the adhesives were applied from a 50% solids solution in toluene and heptane (4 to 1 blend) in a conventional fashion. In all examples and counterexamples, the thickness of the adhesive is around 12 grains/24 in$^2$ (50 microns). For each adhesive, 1% by weight of Irganox™ 1010, a hindered phenol antioxidant available from Ciba-Geigy, was added.

The resulting adhesives had the following compositions (the values in parentheses in Table 1 represent the percent of that particular component in the adhesive composition):

TABLE I

| Ex. | CMTg | Elastomer(s) | Diblock Percent | Liquid Resin/Oil | Solid Resin |
|---|---|---|---|---|---|
| 1 | 254 | Kraton™1112 (52) | 40 | Shellflex™371 ( 2.9) | Wingtack Plus™ (45.1) |
| 2 | 245 | Kraton™1112 (52) | 40 | Shellflex™371 (11.2) | Wingtack Plus™ (36.8) |
| 3 | 264 | Kraton™1112 (30) | 40 | Shellflex™371 (12.8) | Wingtack Plus™ (57.2) |
| 4 | 261 | Kraton™1112 (35) | 40 | Shellflex™371 (11.2) | Wingtack Plus™ (53.8) |
| 5 | 259 | Kraton™1112 (39) | 40 | Shellflex™371 ( 9.5) | Wingtack Plus™ (51.5) |
| 6 | 256 | Kraton™1112 (43) | 40 | Shellflex™371 ( 8.8) | Wingtack Plus™ (48.2) |
| 7 | 252 | Kraton™1112 (47) | 40 | Shellflex™371 ( 9.0) | Wingtack Plus™ (44.0) |
| 8 | 247 | Kraton™1112 (56) | 40 | Shellflex™371 ( 5.9) | Wingtack Plus™ (38.1) |
| 9 | 244 | Kraton™1112 (60) | 40 | Shellflex™371 ( 5.2) | Wingtack Plus™ (34.8) |
| 10 | 247 | Kraton™1112 (38) | 40 | Shellflex™371 (21.4) | Wingtack Plus™ (40.6) |
| 11 | 246 | Kraton™1112 (45) | 40 | Shellflex™371 (16.3) | Wingtack Plus™ (38.7) |
| 12 | 250 | Kraton™1112 (42) | 40 | Shellflex™371 (15.1) | Wingtack Plus™ (42.9) |
| 13 | 255 | Kraton™1112 (35) | 40 | Shellflex™371 (16.5) | Wingtack Plus™ (48.5) |
| 14 | 253 | Kraton™1111 (26) Kraton™1112 (26) | 27.5 | Shellflex™371 ( 4.7) | Wingtack Plus™ (43.3) |
| 15 | 244 | Kraton™1111 (26) Kraton™1112 (26) | 27.5 | Shellflex™371 (12.9) | Wingtack Plus™ (34.1) |
| 16 | 253 | Kraton™1111 (26) Shell RP6403 (26) | 35 | Shellflex™371 ( 4.7) | Wingtack Plus™ (43.3) |

Page 2 of Table I

| Ex. | CMTg | Elastomer(s) | Diblock Percent | Liquid Resin/Oil | Solid Resin |
|---|---|---|---|---|---|
| 17 | 244 | Kraton™1111 (26)<br>Shell RP6403 (26) | 35 | Shellflex™371 (12.9) | Wingtack Plus™ (34.1) |
| 18 | 254 | Kraton™1112 (52) | 40 | Shellflex™371 ( 2.9) | Wingtack Plus™ (45.1) |
| 19 | 245 | Kraton™1112 (52) | 40 | Shellflex™371 (11.2) | Wingtack Plus™ (36.8) |
| 20 | 253 | Kraton™1112 (26)<br>Shell RP6403 (26) | 47.5 | Shellflex™371 ( 3.8) | Wingtack Plus™ (44.2) |
| 21 | 244 | Kraton™1112 (26)<br>Shell RP6403 (26) | 47.5 | Shellflex™371 (12.2) | Wingtack Plus™ (35.8) |
| 22 | 245 | Shell RP6403 (52) | 55 | Shellflex™371 ( 2.9) | Wingtack Plus™ (45.1) |
| 23 | 254 | Shell RP6403 (52) | 55 | Shellflex™371 (11.2) | Wingtack Plus™ (36.8) |
| 24 | 254 | Kraton™1112 (52) | 40 | Shellflex™371 ( 2.9) | Wingtack Plus™ (45.1) |
| 25 | 245 | Kraton™1112 (52) | 40 | Shellflex™371 (11.2) | Wingtack Plus™ (36.8) |
| 26 | 254 | Kraton™1112 (52) | 40 | Zonarez™A-25 ( 5.0) | Escorez™1310 (43.0) |
| 27 | 245 | Kraton™1112 (52) | 40 | Zonarez™A-25 (21.8) | Escorez™1310 (26.2 |
| 28 | 254 | Kraton™1112 (52) | 40 | Shellflex™371 (12.7) | Zonarez™A-135 (35.3) |
| 29 | 245 | Kraton™1112 (52) | 40 | Shellflex™371 (19.2) | Zonarez™A-135 (28.8) |
| 30 | 254 | Kraton™1112 (52) | 40 | ECR™143-H ( 4.0) | Arkon™P100 (44.0) |
| 31 | 245 | Kraton™1112 (52) | 40 | ECR™143-H (19.9) | Arkon™P100 (28.1) |
| 32 | 254 | Kraton™1112 (52) | 40 | Escorez™2520 ( 5.1) | Escorez™1310 (42.9) |

EP 0 558 685 B1

Page 3 of Table I

| Ex. | CMTg | Elastomer(s) | Diblock Percent | Liquid Resin/Oil | Solid Resin |
|---|---|---|---|---|---|
| 33 | 245 | Kraton™1112 (52) | 40 | Escorez™2520 (22.7) | Escorez™1310 (25.3) |
| 34 | 254 | Kraton™1112 (52) | 40 | Escorez™2520 ( 7.6) | Regalite™355 (40.4) |
| 35 | 245 | Kraton™1112 (52) | 40 | Escorez™2520 (24.2) | Regalite™355 (23.8) |

Counterexamples

| Ex. | CMTg | Elastomer(s) | Diblock Percent | Liquid Resin/Oil | Solid Resin |
|---|---|---|---|---|---|
| C1 | 254 | Kraton™1111 (52) | 15 | Shellflex™371 ( 4.7) | Wingtack Plus™ (43.3) |
| C2 | 245 | Kraton™1111 (52) | 15 | Shellflex™371 (12.7) | Wingtack Plus™ (35.3) |
| C3 | 254 | Kraton™1111 (52) | 15 | Shellflex™371 ( 4.7) | Wingtack Plus™ (43.3) |
| C4 | 245 | Kraton™1111 (52) | 15 | Shellflex™371 (12.7) | Wingtack Plus™ (35.3) |
| C5 | 263 | Kraton™1107 (33.5) | 15 | Zonarez™A-25 (20.0) | Escorez™1310 (46.5) |
| C6 | 263 | Kraton™1107 (39) | 15 | Wingtack™10 (10.8) | Wingtack Plus™ (50.2) |
| C7 | 257 | Kraton™1111 (40) | 15 | Shellflex™371 (12.0) | Wingtack Plus™ (48.0) |
| C8 | 255 | Kraton™1111 (38) | 15 | Shellflex™371 (15.3) | Wingtack Plus™ (46.7) |

WJBAPP7/TABLE.I

Kraton™1107 is a polystyrene-isoprene linear block copolymer available from Shell Chemical Co. having a styrene/isoprene ratio of 14/86, approximately 15 to 20 percent diblock (AB) and, 80 to 85 percent triblock (ABA) and a midblock Tg of 215 Kelvin.

Kraton™1111 is a polystyrene-isoprene linear block copolymer available from Shell Chemical Co. having a styrene content of about 21 percent, approximately 15 percent diblock and 85 percent triblock and a midblock Tg of 215 Kelvin.

Kraton™ 1112 is a polystyrene-isoprene linear block copolymer available from Shell Chemical Co. having a styrene content of about 14 percent, approximately 40 percent diblock and 60 percent triblock and a midblock Tg of 215 Kelvin.

Shell™ RP6403 is a polystyrene-isoprene linear block copolymer available from Shell Chemical Co. having a styrene content of about 14 percent, approximately 55 percent diblock and 45 percent triblock and a midblock Tg of 215 Kelvin.

Escorez™ 1310 is a solid $C_5$ tackifying resin available from Exxon Chemical Corp. having a Tg of 313.5 Kelvin.

Wingtack™ Plus is a solid $C_5$ tackifying resin with a Tg of 315 Kelvin available from Goodyear Chemical Co.

Wingtack™ 10 is a liquid $C_5$ hydrocarbon resin with a Tg of 245 Kelvin also from Goodyear Chemical Co.

Zonarez™ A-25 is a liquid alpha pinene tackifying resin with a Tg of 251 Kelvin available from Arizona Chemical Co.

Zonarez™ A-135 is a solid alpha pinene resin with a Tg of 367 Kelvin from Arizona Chemical Co.

Shellflex™ 371 is a naphthenic oil having about 10% aromatics measured by clay-gel analysis having a Tg of 209 Kelvin and is available from Shell Chemical Co.

ECR™ 143H is a hydrogenated aliphatic hydrocarbon resin with a Tg of 247 Kelvin available from Exxon Chemical Corp.

Escorez™ 2520 is a hydrogenated aliphatic hydrocarbon resin with a Tg of 253 Kelvin available from Exxon Chemical Corp.

Arkon™ P100 is a hydrogenated $C_9$ tackifying resin with a Tg of 312.5 Kelvin available from Arakawa Chemical Co.

Regalite™ 355 is a hydrogenated rosin acid with a Tg of 318.1 Kelvin available from Hercules Inc.

The examples were then tested for their shear and 135° peel to a smooth frontal tape surface of biaxially oriented polypropylene (BOPP), and a matte finish cast polypropylene frontal tape, both having an LAB coating. The LAB was a copolymer of vinyl acetate and vinyl alcohol where some of the alcohol groups in the polymer backbone have been reacted with octadecyl isocyanate. The results are depicted in Table II.

TABLE II

| Example | BOPP Shear | BOPP Peel | Shocky | Matte Shear | Matte Peel | Shocky |
|---------|-----------|-----------|--------|-------------|------------|--------|
| 1 | 1400 | 287 (113) | Yes | 1400 | 874 (344) | No |
| 2 | 1400 | 461 (181) | No | 220 | 586 (231) | No |
| 3 | 450 | 213 (84) | Yes | 120 | 662 (261) | Yes |
| 4 | 1150 | 292 (115) | Yes | 220 | 750 (295) | S |
| 5 | 1400 | 342 (135) | Yes | 700 | 714 (281) | S |
| 6 | 1400 | 341 (134) | Yes | 1210 | 656 (258) | No |
| 7 | 1400 | 497 (196) | S | 1040 | 540 (213) | No |
| 8 | 1400 | 425 (167) | No | 1400 | 464 (183) | No |
| 9 | 1400 | 416 (164) | No | 1100 | 390 (154) | No |
| 10 | 40 | 285 (112) | No | 10 | 457 (180) | No |
| 11 | 500 | 471 (185) | No | 76 | 534 (210) | No |
| 12 | 570 | 569 (224) | No | 140 | 619 (244) | No |
| 13 | 240 | 629 (248) | No | 105 | 693 (273) | No |
| 14 | 1400 | 238 (94) | Yes | 1400 | 846 (333) | No |
| 15 | 1150 | 426 (168) | No | 220 | 445 (175) | No |
| 16 | 1400 | 291 (115) | S | 1400 | 920 (362) | No |
| 17 | 990 | 450 (177) | No | 350 | 509 (200) | No |
| 18 | 1400 | 287 (113) | Yes | 1400 | 874 (344) | No |

| | | | | | | |
|---|---|---|---|---|---|---|
| 19 | 1400 | 461 (181) | No | 220 | 586 (231) | No |
| 20 | 1400 | 529 (208) | No | 1400 | 761 (300) | No |
| 21 | 430 | 401 (158) | No | 210 | 620 (244) | No |
| 22 | 405 | 525 (207) | No | 300 | 665 (262) | No |
| 23 | 1400 | 380 (150) | No | 1400 | 847 (333) | No |
| 24 | 1400 | 287 (113) | Yes | 1400 | 874 (344) | No |
| 25 | 1400 | 461 (181) | No | 220 | 586 (231) | No |
| 26 | 1400 | 235 (93) | Yes | 1400 | 825 (325) | No |
| 27 | 370 | 415 (163) | No | 100 | 552 (217) | No |
| 28 | 1400 | 261 (103) | Yes | 1400 | 699 (275) | No |
| 29 | 80 | 463 (182) | No | 16 | 372 (146) | No |
| 30 | 1400 | 281 (111) | Yes | 1400 | 828 (326) | No |
| 31 | 810 | 467 (184) | No | 400 | 548 (216) | No |
| 32 | 1400 | 362 (143) | S | 1400 | 814 (320) | No |
| 33 | 260 | 402 (158) | No | 30 | 541 (213) | No |
| 34 | 1400 | 370 (146) | S | 1400 | 476 (187) | No |
| 35 | 405 | 460 (181) | No | 40 | 268 (105) | No |

## TABLE II continued

| Example | BOPP Shear | BOPP Peel | Shocky | Matte Shear | Matte Peel | Shocky |
|---------|-----------|-----------|--------|-------------|------------|--------|
| C1 | 1400 | 193 (76) | Yes | 1400 | 872 (343) | Yes |
| C2 | 1400 | 448 (176) | S | 210 | 469 (185) | Yes |
| C3 | 1400 | 168 (66) | Yes | 1400 | 801 (315) | Yes |
| C4 | 1400 | 468 (184) | S | 390 | 443 (174) | Yes |
| C5 | 1400 | 145 (57) | Yes | 740 | 545 (215) | Yes |
| C6 | 700 | 179 (70) | Yes | 500 | 630 (248) | Yes |
| C7 | 1400 | 233 (92) | Yes | 1400 | 769 (303) | Yes |
| C8 | 1400 | 432 (170) | S | 60 | 502 (198) | Yes |

S = Semi-shocky

Examples 1-13 demonstrate the effect of CMTg on the performance characteristics of the diaper adhesive fastening tapes. The results of this CMTg variation is depicted graphically in Fig. 1. Line a in Fig. 1 delineates acceptable from unacceptable shear performance to the smooth BOPP film. Below line a, the shear was found to be unacceptable. Below line b, similarly unacceptable shear performance to the cast matte film was encountered. Line c delineates acceptable from unacceptable peel performance. With respect to the smooth BOPP film above line c, the tapes exhibited shocky peels, whereas below line c, the tapes all exhibited smooth or semi-shocky peels. Similarly, line d delineates acceptable versus unacceptable performance with respect to the cast matte film. Thus, overall acceptable performance is between lines a and c for smooth BOPP and between lines b and d for matte cast film for this resin system.

Examples 14 to 23 are examples of the effect of variance of the diblock content of the elastomeric component within the broad preferred range. No significant effects are noticed with the above defined preferred areas I to III when the diblock percentage is varied with the noted exception of examples 20 and 23 which perform better than comparable examples with respect to their peel performance on the smooth BOPP film. This indicates that for higher diblock content adhesives region II provides acceptable performance. Examples 18 and 19 are identical to examples 1 and 2.

Examples 24 to 35 show the effect of varying the tackifying system from the preferred compounding oil and $C_5$ solid tackifier.

Examples 26 and 27 use a liquid alpha pinene resin instead of the oil. This creates a slight decrease in shear and peel performance (more-so shear) which is more noticeable at the lower CMTg formulation (more of the liquid alpha pinene resin).

Examples 28 and 29 use a solid alpha pinene. In this case the more noticeable performance degradation is at the lower CMTg formulation where unacceptable shear is encountered despite these formulations falling within region I.

13

Examples 26-29 appear to indicate that non-preferred alpha pinene and like higher aromatic tackifiers can be used, but possibly only in a blend with more solid tackifiers at lower CMTg values within the preferred range.

Examples 30 and 31 use a liquid aliphatic tackifying resin and a solid hydrogenated $C_9$ tackifying resin. There is no significant performance variation with these particular tackifiers as compared to Examples 24 and 25.

Examples 32 and 33 use a liquid hydrogenated hydrocarbon resin compared to examples 24 and 25. These examples exhibit better peel to smooth film at higher CMTg values within the preferred regions yet unacceptable shear values to cast matte film within the preferred region (region I). This indicates a shift in the useful CMTg values for this particular type of resin.

Examples 34 and 35 also use a hydrogenated rosin acid, however, with no significant additional effects (compared to examples 32 and 33).

### Counterexamples

Counterexamples 1 to 8 demonstrate the effect of lowering the diblock content of the elastomer component. The CMTg values for counterexamples 1 and 3 and 2 and 4 are substantially identical (the variations in properties are minor due to experimental error in measurements or lot variations in the raw materials) and are within the preferred CMTg ranges (254 and 245), yet the peel values are shocky or semi-shocky as compared to otherwise compositionally identical, e.g., examples 1 and 2, which provide non-shocky peels. This demonstrates the effectiveness of high diblock content in reducing shocky peels, while still providing elastomeric compositions with adequate shear performance.

### Test Methods

### 135 Degree Peel from Frontal Tape

The peel adhesion test is a 135 degree peel from a smooth frontal tape surface of biaxially oriented polypropylene(BOPP) or a matte cast polypropylene, having a LAB on it. The peel rate is 12 inches per minute (30 cm. per minute). The tape samples are rolled down onto the frontal tape substrate using two passes of a 4.5 pound (2 kg.) roller. This test is a variation on PSTC-5. The data is reported in grams per inch (grams per cm.) and was run at 70°F (21°C) and 50 percent relative humidity.

### Shear from Frontal Tape Substrate

The shear adhesion is measured by determining the length of time it takes for a 1 inch by 1 inch (2.5 cm. x 2.5 cm.) sample to shear off a frontal tape substrate under a 1 kilogram load. The frontal tape is either a smooth frontal tape(BOPP) with a LAB as described above or a matte polypropylene(PP) with a LAB. The 2 inch by 6 inch (5.1 cm. x 15.2 cm.) piece of frontal tape is laminated to a 2 inch by 6 inch (5.1 cm. x 15.2 cm.) piece of reinforcing tape (DPDY-9377) in order to enhance the stiffness of the substrate. On the side opposite the reinforcing tape, a 1 inch by 2 inch (2.5 cm. x 5.1 cm.) area of the test tape is rolled down onto the frontal tape using 2 passes of a 4.5 pound (2 kg.) roller. The overlap area between the test tape and the substrate is 1 inch by 1 inch (2.5 cm. x 2.5 cm.) The laminated substrate and the test tape are hung vertically in a 40°C oven for 15 minutes after which a 1 kilogram weight is hung from the test tape, generating a shear load at a 180° angle. The time in minutes for the weight to drop is used as the measure of the shear adhesion.

Other embodiments of the invention will be apparent to those skilled in the art from the consideration of the specification of or practice of the invention disclosed herein. It is intended that the specifications and examples be considered as exemplary, with the true scope and spirit of the invention being indicated by the following claims.

### Claims

1. A tape closure system comprising a first closure surface of a substrate having a low adhesion backsize (LAB) coating on the outer face, and a second closure surface of a fastening tape comprising a backing substrate and an adhesive layer on the backing for attachment to the first closure surface outer face, forming the second closure surface, characterized in that said adhesive layer comprises:

(a) 30 to 60 percent of an elastomer component comprised of a block copolymer with A blocks derived primarily from styrene and B blocks derived primarily from isoprene wherein at least 25 percent of the block copolymer is in the form of an AB diblock copolymer,

(b) a solid B black compatible tackifying resin admixed with a liquid B block compatible tackifying resin or a processing oil so as to provide an adhesive composition having a CMTg of from 244 to 265 Kelvin,

which second closure surface adhesive layer on said fastening tape exhibits a shear adhesion of at least about 100 minutes to the LAB coated outer face of said first closure surface and a substantially nonshocky peel at 135 degrees from the outer face of said first closure surface.

2. The taps closure system of claim 1 wherein the elastomer component is further characterized as an ABA block copolymer.

3. The tape closure system of claim 1 characterized in that the tape exhibits a shear adhesion of at least about 200 minutes and a peel adhesion at 135 degrees of at least about 120 grams per cm.

4. The tape closure system of claim 1 characterized in that the tape adhesive composition falls within one of regions I to III of Fig. 1.

5. The tape closure system of claim 1 characterized in that the LAB coated substrate has a smooth surface on the LAB coated outer face, and the tape adhesive composition falls within regions I or III of Fig. 1.

6. The tape closure system of claim 1 characterized in that the LAB coated substrate has a matte surface on the LAB coated outer face, and the tape adhesive composition falls within regions I or II of Fig. 1.

7. The tape closure system of claim 1 characterized in that the LAB coated substrate is a polyolefin diaper frontal tape.

8. A diaper comprising the tape closure system of claim 1.

9. The tape closure systems of claim 5 characterized in that the LAB is a urethane.

10. The tape closure system of claim 6 characterized in that the LAB is a urethane.

**Patentansprüche**

1. Bandverschlußsystem, umfassend eine erste Verschlußoberflache eines Substrats mit einer klebschwachen Rückseitenbeschichtung (LAB) auf der Außenseite und eine zweite Verschlußoberfläche eines Befestigungsbandes mit einem Trägersubstrat und einer Klebstoffschicht auf dem Träger zur Anbringung an der Außenseite der ersten Verschlußoberfläche, die zweite Verschlußoberfläche bildend,
   dadurch gekennzeichnet, daß die Klebstoffschicht umfaßt:
   (a) 30 ... 60 Prozent einer Elastomerkomponente, umfassend ein Blockcopolymer mit hauptsächlich von Styrol abgeleiteten A-Blöcken und hauptsächlich von Isopren abgeleiteten B-Blöcken, wobei mindestens 25 Prozent des Blockcopolymers in Form eines AB-Diblockcopolymers vorliegen;
   (b) einen festen B-Block-kompatiblen, klebrigmachendem Harz, abgemischt mit einem flüssigen B-Blockkompatiblen, klebrigmachendem Harz oder einem Weichmacheröl, um eine Klebstoffzusammensetzung zu schaffen, die eine CMTg von 244 ... 265 Kelvin haben,
   welche Klebstoffschicht der zweiten Verschlußoberfläche auf dem Befestigungsband eine Scherhaftung von mindestens etwa 100 Minuten auf der LAB-beschichteten Außenseite der ersten Verschlußoberfläche aufweist sowie von der Außenseite der ersten Verschlußoberfläche eine weitgehend nichtruckartige Ablösung bei 135 Grad.

2. Bandverschlußsystem nach Anspruch 1, bei welchem die Elastomerkomponente ferner als ein ABA-Blockcopolymer gekennzeichnet ist.

3. Bandverschlußsystem nach Anspruch 1, dadurch gekennzeichnet, daß das Band eine Scherhaftung von mindestens etwa 200 Minuten zeigt und eine Ablösehaftung bei 135 Grad von mindestens etwa 120

EP 0 558 685 B1

g/cm.

**4.** Bandverschlußsystem nach Anspruch 1, dadurch gekennzeichnet, daß die Band-Klebstoffzusammensetzung innerhalb eines der Bereiche I ... III von Fig. 1 liegt.

**5.** Bandverschlußsystem nach Anspruch 1, dadurch gekennzeichnet, daß das LAB-beschichtete Substrat eine glatte Oberfläche auf der LAB-beschichteten Außenseite hat und die Band-Klebstoffzusammensetzung in den Bereichen I oder III von Fig. 1 liegt.

**6.** Bandverschlußsystem nach Anspruch 1, dadurch gekennzeichnet, daß das LAB-beschichtete Substrat eine stumpfe Oberfläche auf der LAB-beschichteten Außenseite hat und die Band-Klebstoffzusammensetzung innerhalb eines der Bereiche I ... III von Fig. 1 liegt.

**7.** Bandverschlußsystem nach Anspruch 1, dadurch gekennzeichnet, daß das LAB-beschichtete Substrat ein Vorderband einer Polyolefin-Windel ist.

**8.** Windel, das Bandverschlußsystem nach Anspruch 1 aufweisend.

**9.** Bandverschlußsystem nach Anspruch 5, dadurch gekennzeichnet, daß das LAB Urethan ist.

**10.** Bandverschlußsystem nach Anspruch 6, dadurch gekennzeichnet, daß das LAB ein Urethan ist.

**Revendications**

**1.** Système de fermeture en ruban comprenant une première surface de fermeture d'un substrat comportant un revêtement d'apprêt de faible adhérence (LAB) sur la surface extérieure et une seconde surface de fermeture d'un ruban de fixation comprenant un substrat de support et une couche d'adhésif sur le support pour l'attachement à la face extérieure de la première surface de fermeture, formant la seconde surface de fermeture, caractérisé en ce que la couche d'adhésif comprend :

(a) 30 à 60 % d'un composant élastomère constitué d'un copolymère bloc avec des blocs A provenant principalement de styrène et des blocs B provenant principalement d'isoprène dans lequel au moins 25 % du copolymère bloc sont sous la forme d'un copolymère dibloc AB,

(b) une résine assurant un collage compatible avec les blocs B solide mélangée à une résine assurant un collage compatible avec les blocs B liquide ou une huile de traitement de manière à obtenir une composition adhésive ayant une CMTg de 244 à 265 Kelvins,

la couche d'adhésif de la seconde surface de fermeture sur le ruban de fixation montrant une adhérence au cisaillement d'au moins environ 100 minutes à la face extérieure revêtue de LAB de la première surface de fermeture et un pelage essentiellement résistant aux chocs à 135 degrés de la surface extérieure de la première surface de fermeture.

**2.** Système de fermeture en ruban suivant la revendication 1, dans lequel le composant élastomère est de plus caractérisé sous la forme d'un copolymère bloc ABA.

**3.** Système de fermeture en ruban suivant la revendication 1, caractérisé en ce que le ruban montre une adhérence au cisaillement d'au moins environ 200 minutes et une adhérence au pelage à 135 degrés d'au moins environ 120 g par cm.

**4.** Système de fermeture en ruban suivant la revendication 1, caractérisé en ce que la composition adhésive du ruban tombe dans une des zones I à III de la figure 1.

**5.** Système de fermeture en ruban suivant la revendication 1, caractérisé en ce que le substrat revêtu de LAB a une surface lisse sur la face extérieure revêtue de LAB et la composition adhésive du ruban tombe dans les zones I ou III de la figure 1.

**6.** Système de fermeture en ruban suivant la revendication 1, caractérisé en ce que le substrat revêtu de LAB a une surface mate sur la face extérieure revêtue de LAB et la composition adhésive du ruban tombe dans les zones I ou II de la figure 1.

16

7. Système de fermeture en ruban suivant la revendication 1, caractérisé en ce que le substrat revêtu de LAB est un ruban frontal pour couche polyoléfinique.

8. Couche comprenant le système de fermeture en ruban de la revendication 1.

9. Systèmes de fermeture en ruban suivant la revendication 5, caractérisés en ce que le LAB est un uréthanne.

10. Système de fermeture en ruban suivant la revendication 6, caractérisé en ce que le LAB est un uréthanne.

**Fiq. 1**